# EUROPEAN PATENT APPLICATION

(11) **EP 4 163 369 A1**
(43) Date of publication of application: **12.04.2023**
(21) Application number: 21201705.7
(22) Date of filing: 08.10.2021
(51) Int. Cl.: C12N 15/10

(54) **USE OF MIXTURES OF POLYVINYLPYRROLIDONE AND AMMONIUM SULFATE IN THE ISOLATION OF NUCLEIC ACIDS**

(71) Applicant: AXAGARIUS GmbH & Co. KG, 52355 Düren (DE)
(72) Inventor: Wyrich, Ralf, 41516 Grevenbroich (DE)
(74) Representative: dompatent von Kreisler Selting Werner - Partnerschaft von Patent- und Rechtsanwälten mbB

(57) **Abstract**

The present invention relates to the inhibitor removal and hence, the improvement of isolation of nucleic acids from crude, inhibitor-rich sample materials by separation methods such as magnetic bead technology. Dedicated inhibitor removal solutions for the isolation of nucleic acids from such sample materials comprising mixtures of polyvinylpyrrolidone and ammonium sulfate suitable for said purpose are provided herewith. Furthermore, provided are nucleic acid separation kits containing such inhibitor removal solutions.

## Description

The present invention relates to the inhibitor removal and hence, the improvement of isolation of nucleic acids from crude, inhibitor-rich sample materials by separation methods such as magnetic bead technology. Dedicated inhibitor removal solutions for the isolation of nucleic acids from such sample materials comprising mixtures of polyvinylpyrrolidone and ammonium sulfate suitable for said purpose are provided herewith. Furthermore, provided are nucleic acid separation kits containing such inhibitor removal solutions.

### Background of the Invention

Different technologies are used for the isolation of nucleic acids (DNA or RNA) from biological samples, ion exchange chromatography, silica membrane technology or magnetic bead technology. Ion exchange chromatography and silica membrane technology are often used as gravity flow protocols or centrifugation protocols to pass a sample lysate, wash and elution buffers through the binding matrix.

In contrast, purification protocols that use magnetic bead technologies are mostly carried out as batch procedures, where beads and sample lysate, wash buffers or elution buffers are mixed for a certain time and the beads are subsequently collected by a magnet and moved to the next extraction step.

The general difference between the two principles is that within the flow through protocols, there is a constant supply of fresh liquid material that passes the chromatography material whereas in the batch procedure, the whole formulation is incubated for a certain time.

In a flow thorough procedure, there is a constant new equilibration between the solid matrix and the passing fluid during the binding step, the washing steps and the elution step. This means, that e.g. during the washing steps fresh wash buffer flows through the solid matrix and efficiently removes contaminants from the matrix, whereas in a batch procedure there is a singular equilibration between the buffer and the solid matrix. Once the equilibration is reached, there is a steady state between desorption and adsorption of contaminating substances from the solid matrix. Thus, regardless how long the matrix is incubated with the fluid, the purity of the matrix will not be further improved.

The basic disadvantage of magnetic bead-based procedures is the reduced efficiency of wash protocols due to the batch procedure. The main advantage of magnetic bead-based extraction procedures is their automation capability. As it is very easy to move magnetic beads between different cavities via a magnet, automation protocols using magnetic beads are wide-spread in the field of molecular biology. The disadvantage of a batch purification needs to be covered with a very efficient chemistry for lysis, binding and washing of the sample material, to generate pure, inhibitor-free eluates. Animal blood samples, which were stored in stabilization solution containing chaotropic salts like guanidinium thiocyanate and guanidinium hydrochloride showed to be problematic in an extraction protocol that was developed for fresh EDTA-blood samples. In contrast to the intended sample material, the stabilized blood samples yielded colored eluates (Example 1), which showed complete inhibition in PCR reactions. During storage in the stabilization solution, the blood sample rendered in such a way, that the extraction protocol was not efficient enough to remove all contaminants.

It is always a problem, if an established extraction method cannot cope with a new sample material, that was not tested during the initial development. Used customers do not want to change their protocols for the established sample materials, therefore a general change of e.g. the wash procedure is not possible, as this would change the general kit concept.

It was therefore necessary to develop a protocol for stabilized blood samples that yielded pure, inhibitor free eluates, which did not change the basic protocol steps of the established protocol and also used the same buffers, so that the established customer base is not forced to re-evaluate the protocol.

State of the art extraction procedures for nucleic acid extraction based on magnetic beads use mixtures of chaotropic salts and alcohols to bind nucleic acids to the beads and also to subsequently wash the beads to purify the nucleic acids. It is also common to add detergents to the solutions or perform enzymatic digestions with proteases to improve the purity of the nucleic acids. A typical extraction procedure based in magnetic bead technology consists of a lysis/binding step, two to three washing steps, normally also a drying step to evaporate alcohol residues and an elution step. For very complex inhibitor-rich samples, standard extraction procedures may not be sufficient to generate clean, inhibitor-free eluates.

On the other hand, polyvinylpyrrolidone (PVP) and ammonium sulfate (AS) are known as additives that are suitable for purifying nucleic acids from biological samples. In particular, CN101935647 describes PVP as a component of a lysis solution and AS as a component of an inhibitor removal solution. The procedure is very complex and needs two precipitation steps to remove the inhibitors. After each precipitation, the sample needs to be centrifuged and the supernatant is processed further. After the second precipitation step, the binding conditions are adjusted, and a binding matrix is added. Moreover, CN102344921 describes PVP as part of a lysis mixture. The sample is cleared via a chloroform extraction and a subsequent centrifugation. The supernatant is then brought into contact with magnetic particles to bind the nucleic acids. A similar use of PVP is described in CN103740700. PVP is part of a lysis mixture, but the removal of contaminants is achieved by a phenol/chloroform extraction. Moreover, the procedure does not use a solid matrix to bind nucleic acids but precipitates the nucleic acid from the supernatant using isopropanol. Furthermore, CN108220286 uses PVP together with ammonium sulfate and guanidine thiocyanate and various surfactants as part of a lysis mixture. The processing of the sample requires vortexing and supernatant transfer steps. US8574890 uses PVP to coat charcoal, which is then used to filter the sample material and remove contaminants. EP0574575 describes the use of PVP to precipitate a complex matrix and isolate biomolecules acids either from the supernatant or the precipitate. EP1044984 describes PVP as part of an extraction buffer in a procedure, where contaminants bind to an adsorption matrix and nucleic acids are again purified from a supernatant.

So far it was unknown that PVP and AS could be used in a batch procedure, where sample lysis and binding of nucleic acids to magnetic beads take place in one batch, without any pre-cleaning steps. The use of a PVP/AS mixture to reduce the co-purification of inhibitory substances from complex sample matrices in a magnetic bead-based batch extraction procedure was not described before.

Thus, there was still a need to improve existing purification protocol for stabilized blood samples that yielded pure, inhibitor free eluates, in which the basic protocol steps of the established protocol need not be changed and which use the same buffers so that the established protocols must not be re-evaluated.

### Short Description of the Invention

The present invention solves the problem of adapting existing protocols to a new challenging sample material without changing the basic principle of the protocols and the basic chemistry involved therewith. As the only way to address the problem of processing inhibitor-rich sample materials without changing the protocol was to find additives for the lysis and binding step, a screening process was started to identify such additives. Surprisingly it was found that a mixture of polyvinylpyrrolidone (PVP) and ammonium sulfate (AS) improved the purity of the eluates and erased PCR inhibitions. The invention thus provides:
(1) A method for isolating nucleic acids from a nucleic acid-containing biological sample, which consists of the following steps:
   (a) providing the nucleic acid-containing sample,
   (b) adding to the sample of step (a) an inhibitor removal solution comprising (1) a polyvinylpyrrolidone (PVP) component containing PVP, and (2) ammonium sulfate (AS) in a volume equal or greater than the volume of the sample;
   (c) loading the sample of step (b) to a magnetic beads separation material to bind the nucleic acids to the separation material;
   (d) separating the beads from the sample by magnetic bead separation and washing the separated magnetic beads with one or more wash buffers to remove unwanted material; and
   (e) releasing the nucleic acids from the separated magnetic beads by applying a release solution.
(2) An inhibitor removal solution as defined in aspect (1) above.
(3) A kit comprising the inhibitor removal solution of aspect (2) above.
(4) The use of the inhibitor removal solution of aspect (2) above for the isolation of nucleic acids from biological samples by magnetic beads separation.

The storage of samples such as blood samples in stabilization solutions that contain guanidinium salts leads to alterations in the sample material, which compromise the quality of isolated RNA and DNA when using standard procedures that were developed for fresh sample materials. This is especially the case for batch procedures using magnetic beads as adsorption matrix. Within a screening process for chemicals that prevent the co-purification of inhibitory substances, it was surprisingly found that solutions of ammonium sulfate and different polyvinyl pyrrolidone species lead to the isolation of pure, inhibitor free RNA and DNA from challenging sample materials. The solutions can easily be implemented into existing protocols, as it can just be added to the lysis and binding mixture. No other changes need to be made to the protocol. An existing customer base can still use the established protocols and add new sample materials. The application is not limited to blood samples but may be used for complex, inhibitor rich biological samples in general.

### Short Description of the Figures

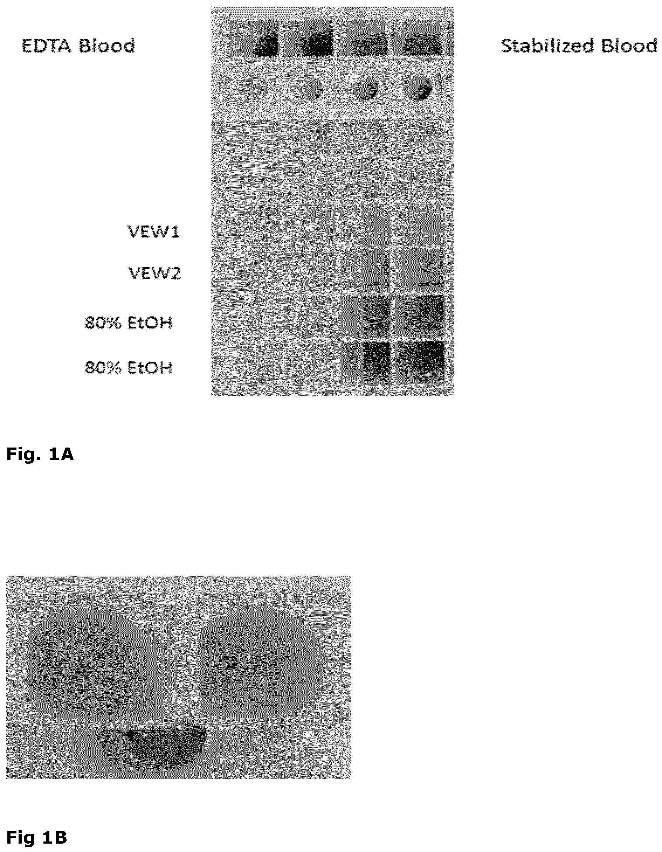

Figure 1: Photograph of the 96-well block (A) and of the eluate (B) of the stabilized sample after the extraction of Example 1.

### Detailed Description of the Invention

Aspect (1) of the invention relate to methods for isolating nucleic acids from a nucleic acid-containing biological sample, which comprise adding to said sample, prior to subjecting it to a separation procedure, a sufficient amount of a specific inhibitor removal solution comprising a polyvinylpyrrolidone (PVP) component and ammonium sulfate (AS), which is hereinafter shortly referred to as the inhibitor removal solution of the invention.

A "nucleic acid-containing biological sample" according to the invention refers to an inhibitor-rich and/or crude nucleic acid-containing biological sample and includes material directly obtained from biological material (such as cells, tissue and bodily fluids) and also synthetic or semi-synthetic material. The nucleic acid-containing may be subjected to lysis of the crude biological material for providing the sample material for the isolation process, including lysing of the material by adding a suitable lysis solution/buffer. Further, "nucleic acids" according to the present invention includes DNA and RNA and derivatives thereof.

Ammonium sulfate is an anti-chaotropic agent, known to precipitate proteins at high concentrations. Poylvinylpyrrolidone is a linear polymer, consisting of vinylpyrrolidone units. Depending on the average chain lengths, PVP can have different molecular weights. The most commonly used PVPs are the following PVPs (with the indicated average MWs in g/mol): PVP K12 (MW 2000-3000); PVP K25 (MW 10.000); PVP K30 (MW 40.000); PVP K90 MW 360.000 as determined by the method described in https://onlinelibrary.wiley.com/doi/abs/10.1002/macp.1952.020070302.

The miscibility of PVP and AS is depending on the MW of the PVP. The higher the MW of PVP is, the lower is the miscibility with AS. Several combinations of the different PVP species and AS were tested.

General design of the experiments: Blood from cattle or sheep was collected in standard EDTA blood collection tubes. An aliquot of the blood was spiked with pathogenic viruses e.g. porcine enterovirus (PEV, contains single stranded RNA as genetic material), canine distemper virius (CDV, contains single stranded RNA as genetic material), bluetongue virus (BTV, contains double stranded RNA as genetic material) or vaccinia virus (POX, contains double stranded DNA as genetic material).

The inhibitor removal solution of the invention comprises the PVP component in an amount of about 2 to about 60 %(w/v), preferably in an amount of about 5 to about 30 %(w/v) and most preferably in an amount of about 8 to about 20 %(w/v). Further, the inhibitor removal solution of the invention comprises the AS in an amount of about 50 mM to about 5 M, preferably in an amount of about 100 mM to about 1.5 M, and most preferably in an amount of about 500 mM to about 1 M. In the inhibitor removal solution of the invention, the weight ratio of PVP component to the AS is from about 10:1 to 1:10, preferably from 5:1 to about 1:5, and most preferably from about 1.5:1 to about 1:4. The inhibitor removal solution may further comprise buffers, salts, chelators, detergents and/or reducing agents.

In the inhibitor removal solution of the invention, the PVP component comprises PVP with an average molecular weight of from about 1000 to about 500000 g/mol, preferably from about 2000 to about 50000 g/mol. Alternatively or additionally the PVP component may comprise alkylated or halogenated derivatives of polyvinylpyrrolidone and/or monomers of the PVP or its derivatives.

In the method of aspect (1), the volume of the inhibitor removal solution added to the sample is equal or greater than the volume of the sample, preferably about three-times the volume of the sample.

The method of aspects (1) comprised in step (c) the loading of the stabilized sample to a magnetic beads separation material to therewith bind the nucleic acids to the separation material. "Magnetic beads separation materials" according to the invention include silica-based materials of various shapes and sizes including silica-based magnetic beads or carboxylated magnetic beads. Prior to or during said loading in step (c), the stabilized sample may further be subjected to a protease digest, e.g. with proteinase K, by adding a protease-containing solution and/or by adding a suitable binding solution. Such binding solution is generally a high salt (sodium perchlorate), buffered (Tris and or citrate buffer), surfactant-containing (Tween^{®} sodium dodecylsulfate (SDS)) and ethanol-containing aqueous solution.

In the method of aspect (1), the wash buffers of step (d) are salt-containing and/or ethanol-containing aqueous solutions (e.g. 0-3 M sodium perchlorate, 80% ethanol 20% water, with 0-10 % (wt/vol) sodium acetate, 0-2 % (wt/vol)Tween^{®} ,0-3 % (wt/vol) sorbitol and 0-9 % (wt/vol) acetic acid). The volume of each wash buffer applied shall be in the range of 10- to 50-fold of the volume of the separation material.

In the method of aspect (1), the release solution is water or an alkaline low-salt buffer (e.g. an aqueous solution containing 5 mM Tris/HCl). The volume of the release solution applied is the 1- to 100-fold of the separation material.

As indicated above, the methods of the invention may the lysis of the sample material, e.g. by mechanical lysis, or by the addition of a distinct lysis buffer and/or lysis solution. Such lysis solution may comprise a lysing enzyme like a protease, the lysis buffer may (further) comprise ionic components, such as salts, chaotropic salts different from those of the stabilizing solution, ionic detergents such as sodium dodecylsulfate (SDS) and/or non-ionic detergents such as Tween^{®}.

In the attached examples, spiked blood samples were mixed with a stabilization solution consisting of two guanidinium salts, guanidinium thiocyanate and guanidinium hydrochloride and stored for at least 18 h at RT or 37 °C. As a reference, the spiked EDTA blood was stored under the same conditions. To compensate for the dilution of the stabilized samples, the EDTA-samples were diluted with lysis buffer to the same extend immediately before extraction.

From all samples 100 µl were used for the extraction. Extractions were performed using the NucleoMag^{®} VET Kit from MACHEREY-NAGEL GmbH & Co.KG (Cat.No. 744200) on a KingFisher^{®} Duo Magnetic Particle Processor (Thermo Fisher Scientific). The EDTA-blood samples were extracted according to the NucleoMag^{®} VET standard protocol (reference 1). An aliquot of the stabilized samples was extracted according to the standard protocol (reference 2). A second aliquot of the stabilized sample was extracted using the PVP/AS solutions.

All extractions were performed in duplicates.

The analysis of the viral RNA was performed using an AgPath-ID^{™} One-Step RT-PCR Kit (Thermo Fisher Scientific Cat.No. 4387424) and specific primers and probes, the DNA from the vaccinia virus was detected using the QuantiTect Multiplex PCR Kit (Qiagen Cat.No. 204543) and specific primers and probe. Real time PCR and RT-PCR was performed on a C1000 Touch^{™}Thermal Cycler and CFX Real Time PCR detection system (BioRad).

The inhibition of the different eluates was evaluated by calculating the delta Cq values between the EDTA (reference 1) and the PVP/AS test samples. The comparison of the EDTA samples and the stabilized samples, extracted with the standard protocol (reference 2) showed the inhibitory effect of the stabilized samples. The comparison of the EDTA samples with the PVP/AS samples showed the reduction of the inhibitory effect due to the addition of PVP/AS.

Preferred embodiments of the stabilizing solution of aspect (2) are those defined for aspect (1) above.

The kit comprising the stabilizing solution and/or the sample collection container of aspect (3) may further comprise one or more of the following components: lysis solutions/buffers, inhibitor removal solutions, washing solutions, release solutions, protease-containing solutions, binding solutions and separation material/beads, including those solutions/buffers/materials specified above.

The invention will be further explained by the following examples, which are not to be construed as limiting the invention.

### Examples

### Abbreviations:

- DNA: desoxyribonucleic acid
- RNA: ribonucleic acid
- PCR: polymerase chain reaction
- RT-PCR: reverse transcriptase PCR
- qPCR: quantitative PCR
- GTC: guanidinium thiocyanate
- GuHCl: guanidinium hydrochloride
- CDV: canine distemper virus
- PEV: porcine enterovirus
- BTV: bluetongue virus
- POX: vaccinia virus
- 6-FAM: 6-carboxyfluorescin--phosphoramidit
- BHQ1: Black Hole Quencher 1
- Cq: quantification cycle
- EtOH: ethanol
- PVP: polyvinyl pyrrolidone
- AS: ammonium sulfate
- MW: molecular weight
- RT: room temperature, about 23 °C

### Materials and Methods:

General design of the experiments: Blood from cattle or sheep was collected in standard EDTA blood collection tubes. An aliquot of the blood was spiked with pathogenic viruses e.g. porcine enterovirus (PEV, raised on pig kidney cells (PK15) with a titer of 10^{5.6} per ml single stranded RNA as genetic material), canine distemper virius (CDV, raised on vero cells with a titer of 10^{3.83} per ml single stranded RNA as genetic material), bluetongue virus (BTV, raised on baby hamster kidney cells (BHK-21) with a titer of 10^{3.2} per ml double stranded RNA as genetic material) or vaccinia virus (POX, raised on vero cells with a titer of 10^{3.5} per ml double stranded DNA as genetic material).

The spiked blood samples were mixed with a stabilization solution consisting of two guanidinium salts, guanidinium thiocyanate (GTC) and guanidinium hydrochloride (GuHCl) and stored for at least 18 h at RT or 37 °C. As a reference, the spiked EDTA blood was stored under the same conditions. To compensate for the dilution of the stabilized samples, the EDTA-samples were diluted with lysis buffer to the same extend immediately before extraction.

From all samples 100 µl were used for the extraction. Extractions were performed using the NucleoMag^{®} VET Kit from MACHEREY-NAGEL GmbH & Co.KG (Cat.No. 744200) on a KingFisher^{®} Duo Magnetic Particle Processor (Thermo Fisher Scientific). The EDTA-blood samples were extracted according to the NucleoMag^{®} VET standard protocol (reference 1). An aliquot of the stabilized samples was extracted according to the standard protocol (reference 2). A second aliquot of the stabilized sample was extracted using the PVP/AS solutions.

All extractions were performed in duplicates.

The analysis of the viral RNA was performed using an AgPath-ID^{™} One-Step RT-PCR Kit (Thermo Fisher Scientific Cat.No. 4387424) and specific primers and probes, the DNA from the vaccinia virus was detected using the QuantiTect Multiplex PCR Kit (Qiagen Cat.No. 204543) and specific primers and probes shown in the following Table 1. Real time PCR and RT-PCR was performed on a C1000 Touch^{™}Thermal Cycler and CFX Real Time PCR detection system (BioRad).

**Table 1**

| Oligonucleotid (SEQ ID NO) | Sequence 5'-3' |
|---|---|
| CDV forward primer (1) | CTG TCR GTA ATC GAG RAT TCG A |
| CDV reverse primer (2) | GCC GAA AGA ATA TCC CCA GTT AG |
| CDV probe (3) | 6-FAM-ATC TTC GCC AGA RTC YTC AGT GCT-BHQ1 |
| BTV forward primer (4) | GTT ACG CAT TAC CGA GGT TGT G |
| BTV reverse primer (5) | GAT CAT GTG TGA ACG CCT TCG |
| BTV probe (6) | 6-FAM-AAC GGC TCA CAC CGA CGA TCC AGC-BHQ1 |
| PEV forward primer (7) | GAA TGC GGC TAA TCC YAA CC |
| PEV reverse primer (8) | GTT GTC ACC ATA AGC AGC CA |
| PEV probe (9) | 6-FAM-ACC CAA AGT AGT CGG TTC CGC C-BHQ1 |
| POX forward Primer (10) | GCC AGA GAT ATC ATA GCC GCT C |
| POX reverse primer (11) | CAA CGA CTA ACT AAT TTG GAA AAA AAG AT |
| POX probe (12) | 6-FAM-TTT TCC AAC CTA AAT AGA ACT TCA TCG TTG CGT T-BHQ1 |

The inhibition of the different eluates was evaluated by calculating the delta Cq values between the EDTA (reference 1) and the PVP/AS test samples. The comparison of the EDTA samples and the stabilized samples, extracted with the standard protocol (reference 2) showed the inhibitory effect of the stabilized samples. The comparison of the EDTA samples with the PVP/AS samples showed the reduction of the inhibitory effect due to the addition of PVP/AS.

Extraction of nucleic acids from EDTA and stabilized samples using NucleoMag^{®} VET Kit. The following protocol was used to isolate nucleic acids from the reference samples 1 and 2 and for example 1:
- 100 µl sample were added to a 96-well deep well block in row A;
- 20 µl proteinase K and 100 µl buffer VL1 were added;
- incubation for 5 min at RT;
- 20 µl NucleoMag^{®} B-beads and 350 µl buffer VEB were added;
- 600 µl wash buffer VEW1, VEW2 and 80% (v/v) ethanol were added to rows F, G and H of the 96-well block;
- 100 µl elution buffer VEL were added to the elution strip: and
- the 96-well block and the elution strip were put onto the appropriate position of a KingFischer Duo processor and the automated extraction protocol was started. Extraction protocol in bief:
- After a 20 min binding step with permanent mixing of the sample, beads were collected by a magnetic rod and transferred into the wash buffer VEW1;
- beads were released again and mixed in VEW1 for 5 min, beads were collected and transferred to the next wash buffer, beads were mixed for 5 min in each washing buffer, after the last washing step with 80% (v/v) ethanol, the beads were air dried for 15 min; and
- beads were transferred to the elution strip, released in buffer VEL and mixed for 5 min in VEL, beads were collected again with the magnetic rod and removed, and the eluted nucleic acid was used for subsequent qPCR analysis.

Extraction of nucleic acids from stabilized blood samples using PVP/AS solutions 1 to 3:
100 µl sample were added to a 96-well deep well block in row A;
- 20 µl proteinase K and 100 µl buffer VL1 were added;
- incubation for 5 min at RT;
- PVP/AS solution was added;
- 20 µl NucleoMag^{®} B-beads and 350 µl buffer VEB were added;
- 600 µl wash buffer VEW1, VEW2 and 80% (v/v) ethanol were added to rows F, G and H of the 96-well block;
- 100 µl elution buffer VEL were added to the elution strip; and the 96-well block and the elution strip were put onto the appropriate position of a KingFischer Duo processor and the automated extraction protocol was started.

Extraction protocol in bief:
- After a 20 min binding step with permanent mixing of the sample, beads were collected by a magnetic rod and transferred into the wash buffer VEW1;
- beads were released again and mixed in VEW1 for 5 min, beads were collected and transferred to the next wash buffer, beads were mixed for 5 min in each washing buffer, after the last washing step with 80% (v/v) ethanol, the beads were air dried for 15 min; and
- beads were transferred to the elution strip, released in buffer VEL and mixed for 5 min in VEL, beads were collected again with the magnetic rod and removed, and the eluted nucleic acid was used for subsequent qPCR analyses.

Extraction of nucleic acids from stabilized blood samples using PVP/AS solution 4
- 100 µl sample + 200 µl PVP/AS solution 4 were added to a 96-well deep well block in row A;
- incubation for 5 min at RT;
- 20 µl proteinase K , 20 µl NucleoMag^{®} B-beads and 350 µl buffer VEB were added;
- 600 µl wash buffer VEW1, VEW2 and 80% (v/v) ethanol were added to rows F, G and H of the 96-well block;
- 100 µl elution buffer VEL were added to the elution strip;
- the 96-well block and the elution strip were put onto the appropriate position of a KingFischer Duo processor and the automated extraction protocol was started. Extraction protocol in bief:
- After a 20 min binding step with permanent mixing of the sample, beads were collected by a magnetic rod and transferred into the wash buffer VEW1;
- beads were released again and mixed in VEW1 for 5 min, beads were collected and transferred to the next wash buffer, beads were mixed for 5 min in each washing buffer, after the last washing step with 80% (v/v) ethanol, the beads were air dried for 15 min; and
beads were transferred to the elution strip, released in buffer VEL and mixed for 5 min in VEL, beads were collected again with the magnetic rod and removed, and the eluted nucleic acid was used for subsequent qPCR analyses.

The following components of the NucleoMag^{®} VET extraction kit were used in the procedures: Lysis buffer VL1; binding solution (buffer) VEB; wash buffers VEW1, VEW2 and 80% (v/v) ethanol, NucleoMag^{®} B-beads, Elution buffer VEL and proteinase K (>2,5 U/mg).

PVP/AS solutions:
PVP/AS solution 1: 9,7% (w/v) PVP K30; 620 mM AS
PVP/AS solution 2: 9,7% (w/v) PVP K25; 620 mM AS
PVP/AS solution 3: 2% (w/v) PVP K90; 600 mM AS
PVP/AS solution 4: 15% (w/v) PVP K12; 800 mM AS

### Example 1: Comparison of the extraction of fresh EDTA- and stabilized blood samples.

Blood from cattle was collected in EDTA-tubes. For stabilization purposes, an aliquot was mixed with a solution containing guanidinium thiocyanate and guanidinium hydrochloride and incubated for several hours. 100 µl of the EDTA blood and the stabilized blood sample was extracted according to the following protocol (duplicate samples) with the NucleoMag^{®} VET Kit:
- 100 µl sample were added to a 96-well deep well block in row A;
- 20 µl proteinase K and 100 µl buffer VL1 were added;
- Incubation for 5 min at RT;
- 20 µl NucleoMag^{®} B-beads and 350 µl buffer VEB were added;
- 600 µl wash buffer VEW1, VEW2 and 2 x 80% (v/v) ethanol were added to rows E, F, G and H of the 96-well block; and
- 100 µl elution buffer VEL were added to the elution strip.

The photographs of the 96-well block and of the eluate of the stabilized sample taken after the extraction are shown in Fig. 1A and B, respectively.

Conclusion: the standard extraction protocol yielded colored eluates with stabilized samples. In contrast to EDTA blood, the wash protocol was not able to remove all contaminants from the magnetic beads.

### Example 2: Extraction of viral RNA from stabilized samples using PVP K25 and AS or K30 and AS.

110 µl of EDTA blood from cattle was spiked with 10 µl of a PBS solution containing PEV. To each stabilized sample, 280 µl of a stabilization solution containing a mixture of guanidinium thiocyanate (GTC) and Guanidinium hydrochloride) (GuHCl) was added. As reference 1, 110 µl of the EDTA blood was spiked with the same amount of PEV virus solution in PBS. All samples were stored for approx. 20 h at RT. Prior to the extraction, the volume of the reference 1 samples was adjusted using 280 µl buffer VL1. For all extractions, 100 µl sample volume were used. Reference 1 samples were extracted using the NucleoMag^{®} Vet protocol. For the stabilized samples, the NucleoMag^{®} Vet protocol was used as reference 2, which represented the inhibited eluates. The stabilized samples were also extracted using the PVP/AS protocol.

Extraction of RNA from EDTA and stabilized samples using NucleoMag^{®} VET Kit:
- 100 µl samples were added to a 96-well deep well block in row A;
- 20 µl proteinase K and 100 µl buffer VL1 were added;
- Incubation for 5 min at RT;
- 20 µl NucleoMag^{®} B-beads and 350 µl buffer VEB were added;
- 600 µl wash buffer VEW1, VEW2 and 80% (v/v) ethanol were added to rows F, G and H of the 96-well block;
- 100 µl elution buffer VEL were added to the elution strip; and
- the 96-well block and the elution strip were put onto the appropriate position of a KingFischer Duo processor and the automated extraction protocol was started. Extraction of RNA from stabilized blood samples using PVP/AS solution:
- 100 µl sample were added to a 96-well deep well block in row A;
- 20 µl proteinase K and 100 µl buffer VL1 were added;
- Incubation for 5 min at RT;
- 120 µl PVP/AS solution 1 or 2 were added;
- 20 µl NucleoMag^{®} B-beads and 350 µl buffer VEB were added;
- 600 µl wash buffer VEW1, VEW2 and 80% (v/v) ethanol were added to rows F, G and H of the 96-well block;
- 100 µl elution buffer VEL were added to the elution strip; and
- the 96-well block and the elution strip were put onto the appropriate position of a KingFischer Duo processor and the automated extraction protocol was started. Detection of virus RNA: Analysis of the RNA was performed on a C1000 Touch^{™}Thermal Cycler and CFX Real Time PCR detection system using AgPath-ID^{™} One-Step RT-PCR Kit and virus specific primers and probes as shown in Table 1. Amplification protocol: 10 min at 45 °C (reverse transcription); 10 min at 95 °C (activation of the Taq-polymerase); 45 cycles: 15 s at 95 °C (denaturation), 30 s at 56 °C (annealing), 30 s at 72 °C (elongation), fluorescence read after each cycle. The results are shown in Table 2.

**Table 2**

| Sample | Cq | Mean | delta Cq/Ref. 1 |
|---|---|---|---|
| Reference 1 | 26,64 | 26,53 | |
| | 26,42 | | |
| PVP/AS solution 1 | 27,09 | 27,16 | 0,62 |
| | 27,22 | | |
| PVP/AS solution 2 | 27,03 | 26,75 | 0,22 |
| | 26,46 | | |
| Reference 2 | 28,07 | 36,54 | 10,00 |
| | 45,00 | | |

The stabilized sample that was extracted using the standard protocol showed strong inhibition. One of the two replicates was completely inhibited, the second replicate showed also a higher Cq value than the reference 1 samples. The average delta Cq compare to reference 1 was 10. In contrast to that, the average delta Cq for PVP/AS solution 1 (PVP K30) was 0,62, the average delta Cq for solution 2 (PVP K25) was 0,22.

Example 3: Extraction of viral RNA from stabilized samples using PVP K90 and AS 150 µl of EDTA blood from sheep was spiked with 15 µl of a PBS solution containing PEV. To each stabilized sample, 400 µl of a stabilization solution containing a mixture of GTC and GuHCl was added. As reference 1, 150 µl of the EDTA blood was spiked with the same amount of PEV virus solution in PBS. All samples were stored for approx. 20 h at 37 °C. Prior to the extraction, the volume of the reference 1 samples was adjusted using 400 µl buffer VL1. For all extractions, 100 µl sample volume were used. Reference 1 samples were extracted using the NucleoMag^{®} Vet protocol. For the stabilized samples, the NucleoMag^{®} Vet protocol was used as reference 2, which represented the inhibited eluates. In addition, the stabilized samples were extracted using the PVP/AS protocol.

Extraction of RNA from EDTA and stabilized samples using NucleoMag^{®} VET Kit:
- 100 µl sample were added to a 96-well deep well block in row A;
- 20 µl proteinase K and 100 µl buffer VL1 were added;
- Incubation for 5 min at RT;
- 20 µl NucleoMag^{®} B-beads and 350 µl buffer VEB were added;
- 600 µl wash buffer VEW1, VEW2 and 80% (v/v) ethanol were added to rows F, G and H of the 96-well block;
- 100 µl elution buffer VEL were added to the elution strip; and
- the 96-well block and the elution strip were put onto the appropriate position of a KingFischer Duo processor and the automated extraction protocol was started. Extraction of RNA from stabilized blood samples using PVP/AS solution:
   - 100 µl sample were added to a 96-well deep well block in row A;
   - 20 µl proteinase K and 100 µl buffer VL1 were added;
   - Incubation for 5 min at RT;
   - 100 µl PVP/AS solution 3 were added;
   - 20 µl NucleoMag^{®} B-beads and 350 µl buffer VEB were added;
   - 600 µl wash buffer VEW1, VEW2 and 80% (v/v) ethanol were added to rows F, G and H of the 96-well block;
   - 100 µl elution buffer VEL were added to the elution strip; and
- the 96-well block and the elution strip were put onto the appropriate position of a KingFischer Duo processor and the automated extraction protocol was started. Detection of virus RNA: Analysis of the RNA was performed on a C1000 Touch^{™}Thermal Cycler and CFX Real Time PCR detection system using AgPath-ID^{™} One-Step RT-PCR Kit and virus specific primers and probes.

Amplification protocol: 10 min at 45 °C (reverse transcription); 10 min at 95 °C (activation of the Taq-polymerase); 45 cycles: 15 s at 95 °C (denaturation), 30 s at 56 °C (annealing), 30 s at 72 °C (elongation), fluorescence read after each cycle. The results are shown in Table 3.

**Table 3**

| Sample | Cq | Mean | delta Cq/Ref. 1 |
|---|---|---|---|
| Reference 1 | 28,71 | 28,82 | |
| | 28,92 | | |
| PVP/AS solution 3 | 30,52 | 30,68 | 1,86 |
| | 30,83 | | |
| Reference 2 | 45,00 | 45,00 | 16,18 |
| | 45,00 | | |

The two replicates from reference 2 showed complete inhibition in the qRT-PCR. In contrast to this, the Cq-increase of the PVP/AS solution 3 (PVP K90) is only moderate, indicating that the eluates were more or less free from inhibitory substances.

Example 4: Extraction of viral RNA from stabilized samples using PVP K12 and AS 150 µl of EDTA blood from cattle was spiked with 15 µl of a PBS solution containing PEV. To each stabilized sample, 400 µl of a stabilization solution containing a mixture of GTC and GuHCl was added. As reference 1, 150 µl of the EDTA blood was spiked with the same amount of PEV virus solution in PBS. All samples were stored for approx. 20 h at RT. Prior to the extraction, the volume of the reference 1 samples was adjusted using 400 µl buffer VL1. For all extractions, 100 µ! sample volume were used. Reference 1 samples were extracted using the NucleoMag Vet protocol. Also for the stabilized samples, the NucleoMag Vet protocol was used as reference 2, which represented the inhibited eluates. In addition, the stabilized samples were extracted using the PVP/AS protocol.

Extraction of RNA from EDTA and stabilized samples using NucleoMag^{®} VET Kit:
- 100 µl sample were added to a 96-well deep well block in row A;
- 20 µl proteinase K and 100 µl buffer VL1 were added;
- Incubation for 5 min at RT;
- 20 µl NucleoMag^{®} B-Beads and 350 µl buffer VEB were added;
- 600 µl wash buffer VEW1, VEW2 and 80% (v/v) ethanol were added to rows F, G and H of the 96-well block;
- 100 µl elution buffer VEL were added to the elution strip; and
- the 96-well block and the elution strip were put onto the appropriate position of a KingFischer Duo processor and the automated extraction protocol was started. Extraction of RNA from stabilized blood samples using PVP/AS solution:
   - 100 µl sample + 200 µl PVP/AS solution 4 were added to a 96-well deep well block in row A;
   - Incubation for 5 min at RT;
   - 20 µl proteinase K , 20 µl NucleoMag^{®} B-beads and 350 µl buffer VEB were added;
   - 600 µl wash buffer VEW1, VEW2 and 80% (v/v) ethanol were added to rows F, G and H of the 96-well block;
   - 100 µl elution buffer VEL were added to the elution strip; and
- the 96-well block and the elution strip were put onto the appropriate position of a KingFischer Duo processor and the automated extraction protocol was started. Detection of virus RNA: Analysis of the RNA was performed on a C1000 Touch^{™}Thermal Cycler and CFX Real Time PCR detection system using AgPath-ID^{™} One-Step RT-PCR Kit and virus specific primers and probes.

Amplification protocol: 10 min at 45 °C (reverse transcription); 10 min at 95 °C (activation of the Taq-polymerase); 45 cycles: 15 s 95 °C (denaturation), 30 s 56 °C (annealing), 30 s 72 °C (elongation), fluorescence read after each cycle. The results are shown in Table 4.

**Table 4**

| Sample | Cq | Mean | delta Cq/Ref. 1 |
|---|---|---|---|
| Reference 1 | 26,31 | 25,92 | |
| | 25,52 | | |
| PVP/AS solution 4 | 25,90 | 25,66 | -0,26 |
| | 25,42 | | |
| Reference 2 | 45,00 | 45,00 | 19,08 |
| | 45,00 | | |

The two replicates from reference 2 showed complete inhibition in the qRT-PCR. The PVP/AS solution 4 samples (PVP K12) showed even slightly lower Cq-values compared to reference 1, indicating eluates, which are completely free of any inhibitors.

### Example 5: Test of the extraction from stabilized blood from different animals using PVP/AS solution 4

Stabilized blood from 8 different animals (3 cattle, 3 goats, 1 sheep and 1 swine) was tested for the extraction of viral RNA and DNA from 4 different viruses (CDV, PEV, BTV and POX).

3 ml EDTA blood from each animal was pipetted into a single 50 ml Falcon tube. 150 µl PBS solution containing all 4 viruses were added to the blood samples and mixed. From each sample, an 1,5 ml aliquot was transferred into a second tube that contained 4 ml stabilization solution consisting of GTC and GuHCl . All tubes were stored at room for 3 days. From each sample 100 µl were used for viral RNA and DNA extraction. As the dilution of the stabilized samples was not compensated for the EDTA samples (Reference 1) the difference between both is a dilution factor of 1: 3,7.

Extraction of RNA from EDTA samples using NucleoMag^{®} VET Kit:
- 100 µl sample were added to a 96-well deep well block in row A;
- 20 µl proteinase K and 100 µl buffer VL1 were added;
- Incubation for 5 min at RT;
- 20 µl NucleoMag^{®} B-Beads and 350 µl buffer VEB were added;
   600 µl wash buffer VEW1, VEW2 and 80% (v/v) ethanol were added to rows F, G and H of the 96-well block;
- 100 µl elution buffer VEL were added to the elution strip; and
- the 96-well block and the elution strip were put onto the appropriate position of a KingFischer Duo processor and the automated extraction protocol was started. Extraction of RNA from stabilized blood samples using PVP/AS solution 4:
   - 100 µl sample + 200 µl PVP/AS solution 4 were added to a 96-well deep well block in row A;
   - Incubation for 5 min at RT
      20 µl proteinase K + 20 µl NucleoMag^{®} B-beads + 350 µl buffer VEB were added;
   - 600 µl wash buffer VEW1, VEW2 and 80% (v/v) ethanol were added to rows F, G and H of the 96-well block;
   - 100 µl elution buffer VEL were added to the elution strip; and
- the 96-well block and the elution strip were put onto the appropriate position of a KingFischer Duo processor and the automated extraction protocol was started. Detection of virus RNA: Analysis of the RNA was performed on a C1000 Touch^{™}Thermal Cycler and CFX Real Time PCR detection system using AgPath-ID^{™} One-Step RT-PCR Kit and virus specific primers and probes as shown in Table 1. Amplification protocol: 10 min at 45 °C (reverse transcription); 10 min at 95 °C (activation of the Taq-polymerase); 45 cycles: 15 s 95 °C (denaturation), 30 s 56 °C (annealing), 30 s 72 °C (elongation), fluorescence read after each cycle. Detection of virus DNA: Analysis of the DNA was performed on a C1000 Touch^{™}Thermal Cycler and CFX Real Time PCR detection system using QuantiTect Multiplex PCR Kit and virus specific primers and probes.

Amplification protocol: 15 min at 95 °C (activation of the Taq-polymerase); 45 cycles: 60 s 95 °C (denaturation), 30 s 60 °C (annealing), 30 s 72 °C (elongation), fluorescence read after each cycle.

The qPCR and qRT-PCR results were evaluated by generating the delta Cq between the EDTA reference and the stabilized samples, extracted using the PVP/AS solution 4. As the dilution factor between the sample types was 1:3,7 , the delta Cq-value between the stabilized and the EDTA samples was expected to be 1,9 (the average Cq-value of the stabilized samples should be 1,9 Cq higher than the average Cq of the EDTA samples if recovery and PCR efficiency are equal).

The Table 5 below shows the mean of the duplicate samples. The mean value for each virus, which represents the mean of 16 samples (8 animals/duplicate samples) is already very close to the theoretical delta Cq of 1.9 (CDV 2.1; PEV 1.8; BTV 1.5; POX 2.1). The total mean of the results for all viruses (mean of 64 analyses) is exactly the expected value of 1.9, indicating that the extraction of viral RNA and DNA from stabilized samples, using the optimized protocol, yielded the same efficiency in qRT-PCR and PCR analyses.

**Table 5**

| CDV | Cattle 1 | Cattle 2 | Cattle 3 | Goat 1 | Goat 2 | Goat 3 | Sheep | Swine | mean |
|---|---|---|---|---|---|---|---|---|---|
| Reference 1 | 30.84 | 30.07 | 27.70 | 29.68 | 29.80 | 30.92 | 30.09 | 30.15 | |
| PVP/AS solution 4 | 32.04 | 33.21 | 30.61 | 32.36 | 31.96 | 30.78 | 32.35 | 32.36 | |
| delta Cq/Ref. 1 | 1.19 | 3.14 | 2.91 | 2.68 | 2.16 | -0.14 | 2.26 | 2.21 | 2.1 |
| | | | | | | | | | |

| PEV | Cattle 1 | Cattle 2 | Cattle 3 | Goat 1 | Goat 2 | Goat 3 | Sheep | Swine | mean |
|---|---|---|---|---|---|---|---|---|---|
| Reference 1 | 29.54 | 28.53 | 28.72 | 28.32 | 27.82 | 29.01 | 28.29 | 28.25 | |
| PVP/AS solution 4 | 30.70 | 30.84 | 31.66 | 30.08 | 29.12 | 30.08 | 29.92 | 30.39 | |
| delta Cq/Ref. 1 | 1.15 | 2.30 | 2.94 | 1.77 | 1.30 | 1.08 | 1.63 | 2.14 | 1.8 |
| | | | | | | | | | |

| BTV | Cattle 1 | Cattle 2 | Cattle 3 | Goat 1 | Goat 2 | Goat 3 | Sheep | Swine | |
|---|---|---|---|---|---|---|---|---|---|
| Reference 1 | 25.83 | 24.46 | 25.16 | 24.41 | 23.63 | 25.42 | 23.58 | 23.44 | |
| PVP/AS solution 4 | 26.52 | 26.08 | 27.01 | 26.16 | 24.83 | 26.12 | 25.17 | 25.67 | mean |
| delta Cq/Ref. 1 | 0.69 | 1.61 | 1.86 | 1.75 | 1.21 | 0.69 | 1.59 | 2.23 | 1.5 |
| | | | | | | | | | |

| POX | Cattle 1 | Cattle 2 | Cattle 3 | Goat 1 | Goat 2 | Goat 3 | Sheep | Swine | |
|---|---|---|---|---|---|---|---|---|---|
| Reference 1 | 32.02 | 32.34 | 30.61 | 32.01 | 32.28 | 32.20 | 31.14 | 32.23 | |
| PVP/AS solution 4 | 33.52 | 34.02 | 32.81 | 34.90 | 33.73 | 33.98 | 34.52 | 34.47 | mean |
| delta Cq/Ref. 1 | 1.50 | 1.68 | 2.20 | 2.89 | 1.44 | 1.78 | 3.38 | 2.24 | 2.1 |
| | | | | | Total mean (all viruses): | | | | 1.9 |

## Claims

1. A method for isolating nucleic acids from a nucleic acid-containing biological sample which consists of the following steps:
(a) providing the nucleic acid-containing sample,
(b) adding to the sample of step (a) an inhibitor removal solution comprising (1) a polyvinylpyrrolidone (PVP) component containing PVP, and (2) ammonium sulfate (AS) in a volume equal or greater than the volume of the sample;
(c) loading the sample of step (b) to a magnetic beads separation material to bind the nucleic acids to the separation material;
(d) separating the beads from the sample by magnetic bead separation and washing the separated magnetic beads with one or more wash buffers to remove unwanted material; and
(e) releasing the nucleic acids from the separated magnetic beads by applying a release solution.

2. The method of claim 1, wherein the inhibitor removal solution contains the PVP component in an amount of about 2 to about 60 %(w/v), preferably in an amount of about 5 to about 30 %(w/v) and most preferably in an amount of about 8 to about 20 %(w/v).

3. The method of claim 1 or 2, wherein the inhibitor removal solution contains the AS in an amount of about 50 mM to about 5 M, preferably in an amount of about 100 mM to about 1.5 M, and most preferably in an amount of about 500 mM to about 1 M.

4. The method of any one of claims 1 to 3, wherein in the inhibitor removal solution the weight ratio of PVP component to the AS is from about 10:1 to 1:10, preferably from 5:1 to about 1:5, and most preferably from about 1.5:1 to about 1:4.

5. The method of any one of claims 1 to 4, wherein the inhibitor removal solution further comprises buffers, salts, chelators, detergents and/or reducing agents.

6. The method of any one of claims 1 to 5, wherein the PVP component comprises
(i) PVP with an average molecular weight of from about 1000 to about 500000 g/mol, preferably from about 2000 to about 50000 g/mol; and/or
(ii) alkylated or halogenated derivatives of polyvinylpyrrolidone.

7. The method of any one of claims 1 to 6, wherein the volume of the inhibitor removal solution added to the sample is about three-times the volume of the sample.

8. The method of any one of claims 1 to 7, wherein the step (a) comprises lysis of biological material for providing the sample, including lysing of the material by adding a suitable lysis solution/buffer.

9. The method of any one of claims 1 to 8, wherein the wash buffers of step (d) are salt-containing and/or ethanol-containing aqueous solutions.

10. The method of any one of claims 1 to 9, wherein the release solution is water or an alkaline low-salt buffer

11. An inhibitor removal solution as defined in any one of claims 1 to 7.

12. A kit comprising the inhibitor removal solution of claim 11.

13. The kit of claim 12 further comprising one or more of lysis solution/buffer, washing solutions, release solution, including those defined in claims 8 to 10, protease-containing solution, binding solution and magnetic beads separation material.

14. Use of the inhibitor removal solution of claim 11 for isolating nucleic acids from biological samples by magnetic beads separation.

## Amended claims

### Amended claims in accordance with Rule 137(2) EPC.

1. A method for isolating nucleic acids from a nucleic acid-containing biological sample which consists of the following steps:
(a) providing the nucleic acid-containing sample,
(b) adding to the sample of step (a) an inhibitor removal solution comprising (1) a polyvinylpyrrolidone (PVP) component containing PVP, and (2) ammonium sulfate (AS) in a volume equal or greater than the volume of the sample;
(c) loading the sample of step (b) to a magnetic beads separation material to bind the nucleic acids to the separation material;
(d) separating the beads from the sample by magnetic bead separation and washing the separated magnetic beads with one or more wash buffers to remove unwanted material; and
(e) releasing the nucleic acids from the separated magnetic beads by applying a release solution.

2. The method of claim 1, wherein the inhibitor removal solution contains the PVP component in an amount of about 2 to about 60 %(w/v), preferably in an amount of about 5 to about 30 %(w/v) and most preferably in an amount of about 8 to about 20 %(w/v).

3. The method of claim 1 or 2, wherein the inhibitor removal solution contains the AS in an amount of about 50 mM to about 5 M, preferably in an amount of about 100 mM to about 1.5 M, and most preferably in an amount of about 500 mM to about 1 M.

4. The method of any one of claims 1 to 3, wherein in the inhibitor removal solution the weight ratio of PVP component to the AS is from about 10:1 to 1:10, preferably from 5:1 to about 1:5, and most preferably from about 1.5:1 to about 1:4.

5. The method of any one of claims 1 to 4, wherein the inhibitor removal solution further comprises buffers, salts, chelators, detergents and/or reducing agents.

6. The method of any one of claims 1 to 5, wherein the PVP component comprises
(i) PVP with an average molecular weight of from about 1000 to about 500000 g/mol, preferably from about 2000 to about 50000 g/mol; and/or
(ii) alkylated or halogenated derivatives of polyvinylpyrrolidone.

7. The method of any one of claims 1 to 6, wherein the volume of the inhibitor removal solution added to the sample is about three-times the volume of the sample.

8. The method of any one of claims 1 to 7, wherein the step (a) comprises lysis of biological material for providing the sample, including lysing of the material by adding a suitable lysis solution/buffer.

9. The method of any one of claims 1 to 8, wherein the wash buffers of step (d) are salt-containing and/or ethanol-containing aqueous solutions.

10. The method of any one of claims 1 to 9, wherein the release solution is water or an alkaline low-salt buffer

11. An inhibitor removal solution as defined in any one of claims 1 to 7.

12. A kit comprising the inhibitor removal solution of claim 11.

13. The kit of claim 12 further comprising one or more of lysis solution/buffer, washing solutions, release solution, including those defined in claims 8 to 10, protease-containing solution, binding solution and magnetic beads separation material.

14. Use of the inhibitor removal solution of claim 11 for isolating nucleic acids from biological samples by magnetic beads separation.
